# EUROPEAN PATENT SPECIFICATION

(11) **EP 1 484 326 B1**
(45) Date of publication and mention of the grant of the patent: **30.04.2008**
(21) Application number: 03705069.7
(22) Date of filing: 12.02.2003
(51) Int. Cl.: C07D 301/32, C07D 301/19, C07D 303/04

(54) **METHOD FOR RECOVERING PROPYLENE OXIDE**
VERFAHREN ZUR GEWINNUNG VON PROPYLENOXID
PROCEDE DE RECUPERATION D'OXYDE DE PROPYLENE

(30) Priority: 15.02.2002 JP 2002038198
(43) Date of publication of application: 08.12.2004
(73) Proprietor: Sumitomo Chemical Company, Limited, Tokyo 104-8260 (JP)
(72) Inventor: TSUJI, Junpei, Ichihara-shi, Chiba 299-0125 (JP); KATAO, Masaaki, Ichihara-shi, Chiba 290-0023 (JP)
(74) Representative: Duckworth, Timothy John
(86) International application number: PCT/JP2003/001421
(87) International publication number: WO 2003/068763

(56) References cited:
- EP-A1- 1 382 601
- WO-A-02/088103
- JP-A- 2003 081 953
- US-A- 5 319 114

## Description

### TECHNICAL FIELD

The present invention relates to a method for recovering propylene oxide. More particularly, the present invention relates to a method for recovering propylene oxide, which is extremely advantageous from the industrial viewpoint in particular, and which can recover propylene oxide in high yield from a liquid reaction mixture obtained and can control problems such as decrease of the yield and corrosion in the purification step of propylene oxide to the minimum level.

### BACKGROUND ART

A method for obtaining propylene oxide by oxidizing propylene using cumene hydroperoxide as an oxygen carrier, is known in WO 01/05778 and US 5 319 114 or the like. In this method, since cumyl alcohol is simultaneously produced together with propylene oxide, it can be repeatedly used by hydrogenating it to convert into cumene and returning to cumene hydroperoxide by oxidizing the cumene.

### DISCLOSURE OF THE INVENTION

An object of the present inventors is to provide a method for recovering propylene oxide, which is extremely advantageous from the industrial viewpoint in particular, and which can recover propylene oxide in high yield from a liquid reaction mixture obtained and can control problems such as decrease of the yield and corrosion in the purification step of propylene oxide to the minimum level.

Namely, the present invention relates to a method for recovering propylene oxide, which comprises separating propylene oxide from a liquid mixture containing propylene oxide, cumene and cumyl alcohol through distillation using a distillation column, wherein the bottom temperature of the distillation column is 190°C or lower, and wherein the liquid mixture containing propylene oxide, cumene and cumyl alcohol is obtained by recovering unreacted propylene from a reaction mixture obtained by subjecting cumene hydroperoxide and propylene to epoxidation.

### BEST MODE FOR CARRYING OUT THE INVENTION

In the present invention, the liquid mixture containing propylene oxide, cumene and cumyl alcohol is obtained by subjecting cumene hydroperoxide and propylene to epoxidation in the presence of a catalyst. Further, the epoxidation is preferably carried out in the presence of a catalyst containing titanium-containing silicon oxide from the viewpoint of obtaining propylene oxide in high yield and high selectivity. These catalysts are preferably so-called titanium-silica catalysts containing titanium chemically bonded to silicon oxide. For example, a catalyst prepared by supporting a titanium compound on a silica carrier, a catalyst prepared by combining a titanium compound with silicon oxide by a co-precipitation method or sol gel method, a zeolite compound containing titanium, or the like, can be listed.

Cumene hydroperoxide used as a raw material for epoxidation is usually obtained by auto-oxidation using an oxygen-containing gas such as air, oxygen-concentrated air or the like, and may be a dilute or dense purified material or non-purified material. The solvent may be that which is composed of a material present in a cumene hydroperoxide solution used. But, it is preferable from the industrial viewpoint to use cumene as the solvent without adding a solvent in particular.

The epoxidation temperature is usually from 0 to 200°C, and preferably from 25 to 200°C. The pressure is usually 100 to 20000 kPa, but a pressure of 100 to 10000 kPa is preferable considering the reaction temperature and economical efficiency.

The epoxidation can be carried out advantageously using a catalyst in the form of slurry or fixed bed. In the case of a large-scale industrial operation, a fixed bed is preferably used.

The amount of propylene is usually 1 to 20 times by mole, preferably 5 to 20 times by mole to a mole amount of fresh cumene hydroperoxide supplied for epoxidation. It is effective on maintenance of high level of propylene oxide yield to use an excess amount of propylene to fresh hydroperoxide. The excess amount of propylene left after the epoxidation is usually recovered by distillation.

In the present invention, recovery of propylene oxide is conducted by distillation. The recovery of propylene oxide is carried out using a liquid mixture containing propylene oxide, cumene and cumyl alcohol after recovery of unreacted propylene when energy required for distillation is considered. Though distillation conditions varies depending on the temperature and composition of a liquid reaction mixture supplied, a pressure of 0 to 1 MPa, preferably 0 to 0.1 MPa in terms of a gauge pressure and a temperature of the top of the column of 0 to 100°C are usually illustrated. As the distillation method, a packed column method using packing, and a plate column method using sieve trays or the like are illustrated, and the packed column method is preferable from the viewpoint of controlling the pressure difference between the bottom and top of the column to low, and the bottom temperature to low. Preferably, the distillation column is a packed column.

In the present invention, it is required for achieving the object of the present invention that the bottom temperature of the distillation column is 190°C or lower while propylene oxide is recovered. Besides, there is not the lower limit of the bottom temperature in particular, but the lower limit is preferably about 100°C. Preferably. the bottom temperature of the distillation column in from 100 to 190°C.

Because the concentration of cumyl alcohol has become high after removal of unreacted propylene, generation of water caused by dehydration of cumyl alcohol becomes remarkable when the bottom temperature becomes higher than 190°C, therefore, large energy for distillation separation in a propylene oxide purification step as the next step, becomes necessary. Further, water generated forms glycols by reaction with propylene oxide, and decreases the yield of propylene oxide. In some cases, corrosion of materials of a purification plant caused by formation of condensed water, is apprehended.

### EXAMPLE

The present invention will be illustrated in more detail by using Examples below.

### Example 1

40 Grams of a mixed solution composed of 5% by weight of cumyl alcohol and 95 % by weight of cumene as a raw material were charged in an autoclave, then stirred and heated at 136°C for 60 minutes. α-Methyl styrene was produced by dehydration of cumyl alcohol. The result is shown in Table 1.

### Example 2

It was carried out in the same manner as in Example 1 except that the temperature is 155°C. The result is shown in Table 1.

### Example 3

It was carried out in the same manner as in Example 1 except that the temperature is 184°C. The result is shown in Table 1.

### Comparative Example 1

It was carried out in the same manner as in Example 1 except that the temperature is 194°C. The result is shown in Table 1.

**Table 1**

| | Example 1 | Example 2 | Example 3 | Comparative Example 1 |
|---|---|---|---|---|
| Temperature heated (°C) | 136 | 155 | 184 | 194 |
| α-MS yield(%) *1 | 0.1 | 1.3 | 3.5 | 11.8 |

| | | | | |
|---|---|---|---|---|
| * 1: α-Methyl styrene yield (%) = [Amount of α-methyl styrene produced(mol)/amount of cumyl alcohol in raw material(mol)] x 100 | | | | |

The above Examples and Comparative Example were carried out by using the raw material described above as a bottom liquid, respectively, and it is found that when the temperature heated became 190°C or higher, production of α-methyl styrene, in other words, production of water was remarkable.

### Example 4

40 Grams of a cumene mixed solution containing 3% by weight of propylene oxide and 24% by weight of cumyl alcohol were charged in an autoclave, then stirred and heated at 136°C for 60 minutes. α-Methyl styrene was produced by dehydration of cumyl alcohol. Further, propylene glycol was produced through a reaction of water with propylene oxide. The result is shown in Table 2.

### Example 5

It was carried out in the same manner as in Example 4 except that the temperature is 155°C. The result is shown in Table 2.

### Example 6

It was carried out in the same manner as in Example 4 except that the temperature is 174°C. The result is shown in Table 2.

### Comparative Example 2

It was carried out in the same manner as in Example 4 except that the temperature is 194°C. The result is shown in Table 2.

**Table 2**

| | Example 4 | Example 5 | Example 6 | Comparative Example 2 |
|---|---|---|---|---|
| Temperature heated (°C) | 136 | 155 | 174 | 194 |
| α-MS yield(%) | 0.01 | 0.02 | 0.07 | 0.40 |
| PG concentration (ppm by weight) *2 | 18 | 35 | 42 | 77 |

| | | | | |
|---|---|---|---|---|
| *2: Concentration of propylene glycol in mixture after heating | | | | |

In each of the above Examples 4-6 and Comparative Example 2, a liquid in which a small amount of propylene oxide was added to cumyl alcohol and cumene, was used, and it is found that when the temperature heated became 190°C or higher, production of α-methyl styrene remarkably increases, and propylene glycol also increases together with the increases of α-methyl styrene.

### INDUSTRIAL APPLICABILITY

As described above, according to the present invention, there can be provided a method for recovering propylene oxide, which is extremely advantageous from the industrial viewpoint in particular, and which can recover propylene oxide in high yield from a liquid reaction mixture obtained and can control problems such as decrease of the yield and corrosion in the purification step of propylene oxide to the minimum level.

## Claims

1. A method for recovering propylene oxide, which comprises separating propylene oxide from a liquid mixture containing propylene oxide, cumene and cumyl alcohol through distillation using a distillation column, wherein the bottom temperature of the distillation column is 190°C or lower and wherein the liquid mixture containing propylene oxide, cumene and cumyl alcohol is obtained by recovering unreacted propylene from a reaction mixture obtained by subjecting cumene hydroperoxide and propylene to epoxidation.

2. A method according to claim 1, wherein the bottom temperature of the distillation column is from 100 ° C to 190°C.

3. A method according to either claim 1 or claim 2, wherein the distillation column is a packed column

## Patentansprüche

1. Verfahren zur Gewinnung von Propylenoxid, umfassend Trennen von Propylenoxid von einem flüssigen Gemisch, welches Propylenoxid, Cumol und Cumylalkohol enthält, durch Destillation unter Verwendung einer Destillationssäule, wobei die Sumpftemperatur der Destillationssäule 190 °C oder weniger beträgt und wobei das flüssige Gemisch, welches Propylenoxid, Cumol und Cumylalkohol enthält, erhalten wird durch Rückgewinnung von nicht-umgesetztem Propylen aus einem Reaktionsgemisch, das erhalten wird, indem Cumolhydroperoxid und Propylen einer Epoxidierung unterzogen werden.

2. Verfahren nach Anspruch 1, wobei die Sumpftemperatur der Destillationssäule 100 °C bis 190 °C beträgt.

3. Verfahren nach Anspruch 1 oder Anspruch 2, wobei die Destillationssäule eine gepackte Säule ist.

## Revendications

1. Procédé pour récupérer de l'oxyde de propylène qui comprend la séparation de l'oxyde de propylène d'un mélange liquide contenant de l'oxyde de propylène, du cumène et de l'alcool cumylique par distillation au moyen d'une colonne de distillation, où la température de fond de la colonne de distillation est 190°C ou moins et où le mélange liquide contenant de l'oxyde de propylène, du cumène et de l'alcool cumylique est obtenu en récupérant le propylène qui n'a pas réagi d'un mélange réactionnel obtenu en soumettant de l'hydroperoxyde de cumène et du propylène à une époxydation.

2. Procédé selon la revendication 1, où la température de fond de la colonne de distillation est de 100°C à 190°C.

3. Procédé selon la revendication 1 ou la revendication 2, où la colonne de distillation est une colonne à garnissage.
